# EUROPEAN PATENT SPECIFICATION

(11) **EP 1 218 063 B1**
(45) Date of publication and mention of the grant of the patent: **14.04.2004**
(21) Application number: 00951845.7
(22) Date of filing: 21.07.2000
(51) Int. Cl.: A61P 17/02, A61P 17/12, A61K 31/19

(54) **PHARMACEUTICAL COMPOSITION CONTAINING TRICHLOROACETIC ACID, 2-OH BENZOIC ACID AND MENTHOL FOR TOPICAL APPLICATION AND PROCESS FOR THE PREPARATION THEREOF**
PHARMAZEUTISCHE ZUSAMMENSETZUNG ENTHALTEND TRICHLOROESSIGSÄURE, 2-OH BENZOESÄURE UND MENTHOL ZUR TOPISCHEN ANWENDUNG UND VERFAHREN ZU DEREN HERSTELLUNG
COMPOSITION PHARMACEUTIQUE COMPRENANT ACIDE TRICHLORACETIQUE, 2-OH ACIDE BENZOIQUE ET MENTHOL A USAGE TOPIQUE ET SON PROCEDE DE PREPARATION

(30) Priority: 21.07.1999 IT RM990465
(43) Date of publication of application: 03.07.2002
(73) Proprietor: STRUMILLO, Maria, I-00162 Roma (IT)
(72) Inventor: STRUMILLO DJACZENKO, Maria, I-00162 Roma (IT); FAVA, Danila, I-00141 Roma (IT); DJACZENKO, Wiktor, DECEASED (IT)
(74) Representative: Taliercio, Antonio
(86) International application number: PCT/IT2000/000309
(87) International publication number: WO 2001/005387

(56) References cited:
- EP-A- 0 059 441
- US-A- 4 902 501
- US-A- 5 043 357
- NGUYEN Q.H. ET AL: "Management of acne vulgaris." AMERICAN FAMILY PHYSICIAN, (1994) 50/1 (89-96). , XP000986590

## Description

The present invention relates to pharmaceutical compositions for topical use comprising a mixture, in the suitable polymer, of trichloroacetic acid, 2-hydroxybenzoic acid, (1α, 2β, 5α)-5-methyl-2-(1-methylethyl)cyclohexanol and, if desired, other pharmaceutically acceptable adjuvants and excipients and a process for the preparation thereof.

More particularly the invention relates to pharmaceutical compositions for the treatment of cutaneous injuries as, for example, those resulting from mechanical traumas or surgical operations, burns, dermatitis both from animal sting and animal or poisonous plant contact, for the prevention and therapy of keloids and hypertrophic cicatrices like outcomes from said cutaneous injuries and for the use in aesthetic medicine.

Keloids are connective neoformations occurring by an altered cutis healing following surgical injuries, traumas, burns (Moschella and Hurley, Eds.: Dermatology; W.B. Saunders Company). They appear as pinkish/red cords or plaques, being painful and pruriginous, with variable size and secondary branches invading surrounding tissues. They can be so extended and/or located to result in remarkable aesthetic damages and/or functional limitation of the involved body segment. Their growth rate is rather variable till to a defined size, never spontaneously regressing. The pathogenesis up to now is not well clear: from an histological point of view there is a remarkable inflammatory reaction with an excessive dermal deposition of collagen by fibroblasts.

The hypertrophic cicatrices have origin and appearance similar to keloids differentiating therefrom by the lacking of branches penetrating the healthy surrounding cutis; those deriving from I and II degree burns tend to regress, determining often functional modifications of the affected region. The natural regression is uncommon and the treatable injuries usually do not recidivate.

Various methods are used for the therapy of the hypertrophic cicatrices and keloids: surgical excision, cryotherapy, radiotherapy, lasertherapy, compressive bandages, silicone sheets, and intrainjury injections containing steroids or interferon (both α-IFN and γ-IFN, 2b). Almost all these methods are painful and the less painful thereof show other drawbacks, like restrictions in usual daily activities of patients (compressive bandages) or local side-effects (silicone sheets). It is noteworthy to point out that a significant amelioration of the injury, based on the evaluation of the symptomatology, appearance and consistency thereof, is reached mainly by combination of various methodologies (for example: surgery or cryotherapy combined with intrainjury injections containing steroids), with a consequent greater discomfort for the patient. Furthermore some of these treatments are burdened by a high percentage of recurrences, often more extended than the initial formations (the surgical excision induces recurrences in 45-100 % of the cases) (Berman B. et al., Eur. J. Dermatol. 1998 Dec.: 8(8): 591-5). Finally, due to the intrinsic limitations of each method, no one of the above therapies can be applied at the same time to the several body segments wherein keloids or hypertrophic cicatrices deriving from large burns are present.

The prevention of the hypertrophic cicatrices or keloids following traumas or surgical operations is even less satisfying, not being justified the use of too aggressive methodologies in the prevention of the injuries.

The dermatitis resulting from the contact with plants like *Uetica*, *Euphorbia, Toxidendron, Primula*, is usually treated both topically and systemically by anti-inflammatory, analgesic and/or anti-histamine drugs. Any kind of therapy does not clear up in short times the cutaneous injury, which, where resulting from Toxidendron, can last months too. Also dermatitis from stings of toxic Arthropoda, wasp, hornet, bee, ant or spider, notwithstanding topically or systemically treated, can last from at least 5 days to a few week. The conventional medicine suggests the use of anti-histamine, anti-inflammatory and/or analgesic drugs and topical application of papain. At last some marine toxic invertebrates belonging to Coelenterata bring about a severe, very painful, contact dermatitis. The conventional medicine suggests the topical use of 4-6-% acetic acid. For all the above sting or contact dermatitis the conventionally used treatment does not provide a quick resolution of local signs and symptoms and the therapeutic approach often involves the use of more than one drug. (Oxford Textbook of Medicine; edited by D.J. Weatherall, J.G.G. Ledingham and D.A. Warrell).

The burns are pathological alterations of the tissues as a result of the action of heat, chemical compounds or ultraviolet rays. They are classified based on the damage degree as 1^{st} (epidermal), 2^{nd} (dermal) and 3^{rd} (sub-dermal) degree burns. In the 1^{st} degree and 2^{nd} superficial degree burns, respectively characterised by topical erythema and pain (1°) and erythema, pain and appearance of phlyctena (2° superficial), the resolution of the injury usually occurs within 5-14 days and the topical therapy is based on disinfectants, grease impregnated gauze and, if the case, antibiotics. In the 2^{nd} deep and 3^{rd} degree burns, characterised by the necrosis of the involved tissues, the pain can be not intense due to the destruction of the nervous terminations and the topical pharmacological treatment, using silver nitrate and antibiotics, aims principally to prevent the infections. The 2^{nd} deep and 3^{rd} degree burns always result in keloids and/or regressing hypertrophic cicatrices.

Accordingly it is therefore clear the need to provide new products suitable to resolve the above described problems.

The authors of the present invention surprisingly found out that the topical application of a composition comprising a mixture, in the suitable polymer, of trichloroacetic acid, 2-hydroxybenzoic acid (salicylic acid), (1α, 2p, 5α)-5-methyl-2-(1-methylethyl)cyclohexanol (menthol) and, if desired, other pharmaceutically acceptable adjuvants and excipients is able to reduce dramatically and, in many cases, to result in a complete disappearance of the hypertrophic cicactrices and keloids. It is important to point out that these results are obtained and maintained also after the end of the therapy without other associated treatments.

Another advantage of the title composition is that its topical application is completely painless and does not result in any discomfort for the patient being treated. This characteristic is extremely important in order to reach the patient compliance, especially if children, often affected by burns, are involved.

Furthermore said composition in the form of liquid, gel or ointment allows any body segment, also where seat of joint, to be treated without any limitation for the quotidian life of the patient. These properties, associated with the complete absence of discomfort for the patient, make the title composition the ideal therapy to treat at the same time extended body surfaces, as it is needed in the case of patients affected by keloids and hypertrophic cicatrices as outcomes of large burns.

Finally the treatment is suitable both for the prevention and therapy of said cutaneous injuries.

Furthermore it was surprisingly found out that the topical application of said composition for the treatment of burns, dermatitis resulting both from animal sting and animal or poisonous plant contact, provides a quick amelioration of the sings and symptoms without the need of the administration of other drugs.

The individual components of said composition are already known in medical field but they are used in the form of topical application for objectives different from those above reported.

Salicylic acid, which provides antipyretic, anti-inflammatory and analgesic activity, is used topically for the treatment of calli, verrucae and aphthae of the oral- mucosa. Menthol provides anti-pruriginous activity and it is used in the topical compositions also in order to enhance the permeation of active principles through the skin. Trichloroacetic acid is used in aesthetic medicine for the chemical peeling treatments.

Said compounds, both alone and combined, up to now have not been used for the treatments of cutaneous pathologies, like those above reported, resulting from mechanical traumas or surgical operations, burns, dermatitis both from animal sting and animal or poisonous plant contact, keloids, hypertrophic cicatrices. Furthermore it was unpredictable that combinations of irritant compounds like trichloroacetic acid and salicylic acid could be applied on cutis injured by burns or dermatitis without generating pain, but rather accelerating the regression of symptomatology or on keloids or hypertrophic cicatrices, particularly resistant to any therapy, obtaining a significant volume decrease or disappearance of the injuries.

As above mentioned is already known in aesthetic medicine the treatment called "chemical peeling", principally used to reduce the cutaneous signs of ageing. It is known that as the age proceeds the exchange rate of the cutaneous cells decreases and irregular thickenings of the surface layer of the cutis are created. These alterations generate rugae, principally in the regions subjected to surface tensional stresses as, for example, the regions surrounding the mouth, eyes, neck. The chemical peeling aims to remove the more superficial layers of the cutis and stimulate the regeneration, followed by the amelioration of the appearance of the skin. In addition it is used for the treatment of cicatrices from acne, deep hyper-pigmented spots, also of post-inflammatory origin, and seborrhoeic keratosis.

Based on the kind of used substances and concentrations thereof it is possible to obtain a surface or middle depth exfoliation.

In order to reduce the cutaneous signs of the ageing by a middle depth exfoliation the Jessner's solution, resorcin paste and trichloroacetic acid are used, for example, while glycolic acid is used as superficial exfoliating agent.

Trichloroacetic acid, usually used at a 35 % concentration to reduce rugae and cutaneous spots, can cause local side-effects which can include erythematous reactions and moderate cutaneous superficial sores, thus requiring the treated subject to stay at home for a few days. Furthermore has been disclosed its sequential association with other substances and/or methodologies (vitamin A, glycolic acid, Jessner's solution, Dermasanding, CO₂ laser, hydrocortisone) for a rejuvenation treatment of the neck cutis globally lasting about 9 weeks (Fulton JE et al., Dermatol. Surg 1999 Oct; 25 (10): 745-750) or with the Jessner's solution for the treatment of the actinic keratosis (Witheiter DD et al., Dermatol Surg 1997 Mar; 23(3): 191-6).

Recently the chemical peeling by using 20-30 % salicylic acid has been also suggested to reduce the small rugae and cutaneous hyperpigmentations in subjects having type V or VI cutis (Grimes PE, Dermatol Surg 1999 Jan: 25(1):18-22).

The authors of the present invention found out that said composition comprising a mixture, in the suitable polymer, of trichloroacetic acid, salicylic acid, menthol at suitable concentrations and, if desired, other pharmaceutically acceptable adjuvants and excipients, can be advantageously used to carry out the chemical peeling without generation of any side-effect as, for example, rubefaction, sores, cutaneous spots.

It is therefore an object of the present invention a pharmaceutical composition for topical use comprising a mixture, in the suitable polymer, of trichloroacetic acid, 2-hydroxybenzoic acid, (1α, 2β, 5α)-5-methyl-2-(1-methylethyl)cyclohexanol and, if desired, other pharmaceutically acceptable adjuvants and excipients. Examples of polymers which can be used in said composition are polyethylene glycols, among which tetraethylene glycol. and hexaethylene glycol, polyethoxy ethylene alcohol. As adjuvants and excipients sodium chloride, ethanol can be used. The concentration of trichloroacetic acid can be from about 20 % to about 45 % w/v, preferably it is used at a concentration of about 25 % w/v. The content of 2-hydroxybenzoic acid is from about 10 % to about 30 % w/v, preferably about 20 % w/v.

The composition according the invention, if desired, can also comprise one or more corticosteroids like, for example, triamcinolone, betamethasone, methylprednisolone and dexamethasone.

Triamcinolone can be used at concentrations from about 0,05 % to about 20 %, preferably from about 0,1 % to about 10 %.

The present invention makes available the use of the composition for the treatment of cutaneous injuries as those resulting from mechanical traumas or surgical operations, burns, dermatitis both from animal sting and animal or poisonous plant contact.

Furthermore the invention makes available the use of the composition for the therapy and prevention of the hypertrophic cicatrices and keloids as outcomes of said cutaneous injuries. According to a further aspect of the invention said composition can be advantageously used in aesthetic medicine, for example as exfoliating preparation.

Said composition can be used in various forms as, for example, ointment, foam, liquid preparation or medicated plaster.

A further object of the present invention is represented by the process for the preparation of the pharmaceutical composition comprising the following steps: a) prepare, in an anhydrous atmosphere, the distinct A and B mixtures, respectively, of (A) trichloroacetic acid at a concentration from about 40 % to about 90 % in the suitable polymer and (B) 2-hydroxybenzoic acid at a concentration from about 20 % to about 60 %, in the suitable polymer; b) mix, by small subsequent aliquots, same volumes of the two mixtures to obtain the A+B mixture; c) add a volume of a (1α, 2β, 5α)-5-methyl-2-(1-methylethyl)cyclohexanol saturated solution in anhydrous ethanol equal to 2 % of the volume of the A+B mixture; d) add sodium chloride in a such amount to obtain a final concentration of about 1,2 % w/v; e) keep the thus obtained composition in a bottle filled with anhydrous air at 30°C in reduced pressure conditions and leave at ambient temperature for about 24 hours.

Particularly, the polymer used in the preparation of the A and B mixtures is selected from the group consisting of polyethylene glycols, among which tetraethylene glycol, hexaethylene glycol, polyvinyl alcohol, polyoxyethylene glycol. One or more corticosteroid like, for example, triamcinolone, betamethasone, methylprednisolone and dexamethasone can be added, if desired, to the mixture obtained in step d) above. Particularly, triamcinolone is added in a such amount to obtain a final concentration in the range from about 0,05 % to about 20 %, preferably from about 0,1 % to about 10 %.

The present invention will be now described, by way of illustrative but not limiting examples, according to preferred embodiments thereof.

### Example 1

### Process for the preparation of the composition comprising about 25 % of trichloroacetic acid and about 20 % of 2-hydroxybenzoic acid

A mixture A containing trichloroacetic acid in hexaethylene glycol (apparent molecular weight 290,0) at a 50 % w/v concentration in anhydrous atmosphere (in a chemical hood in the presence of phosphorus pentoxide) was prepared, by adding the acid to the polymer portionwise and under continues stirring to enhance the mixing.

Further a mixture B containing 2-hydroxybenzoic acid (as granules having diameter lower than 50 µm) in hexaethylene glycol (apparent molecular weight 290,0) at a 40 % w/v concentration (in a chemical hood in the presence of phosphorus pentoxide) was prepared. The acid was added to the polymer portionwise and under continuos stirring to enhance the mixing.

After the preparation of said mixtures same volumes thereof were mixed to obtain a 50 % v/v A+B mixture.

The mixture was prepared using a 1000 ml tightly closed glass container (Schott) on whose bottom 10 ml of the A mixture were poured. Then 1 ml of the mixture B and component A, respectively, were stratified in the order. Said amounts of A and B were again poured alternatively layer by layer to a 990 ml volume upon which the filling of the container was completed by pouring 10 ml of mixture B.

A volume of a (1α, 2β, 5α)-5-methyl-2-(1-methylethyl)cyclohexanol saturated solution in anhydrous ethanol equal to 2 % of volume of the A+B mixture was added; subsequently sodium chloride was added in a such amount to obtain a final concentration of about 1,2 % w/v. Anhydrous air (through a solution of concentrated sulphuric acid) was blown within the thus obtained composition at a temperature of 30°C and reduced pressure, till air bubbles gurgled uniformly thorough the container, followed by the closing thereof.

The composition was subsequently left on standing at ambient temperature over 24 hours before the use.

### Example 2

### Results of the topical treatment of keloids and hypertrophic cicatrices by the composition prepared according to example 1

The pharmaceutical composition according to the invention was evaluated by a case study of n° 50 subjects, among which n° 15 were affected both by keloids and hypertrophic cicatrices (outcomes of burns), n° 25 only by keloids (post-traumas or post-surgical operations) and n° 10 only by hypertrophic cicatrices (post-traumas or post-surgical operations). The treatment was consisting of topical applications of the composition in a liquid form, by using a soft brush, with a frequency variable from thrice to once a week, depending on the patient and severity of the injury to be treated. After four weeks of treatment all the subjects related about a remarkable decrease of the itchiness and tenderness of the keloids and a significant decrease of the rubefaction both of the hypertrophic cicactrices and keloids. Furthermore the injuries appeared softer when touched. In the following steps of the treatment all the patients early showed disappearance of the itchiness, tenderness and rubefaction of the injuries and subsequently a remarkable reduction of the volume thereof. The keloids required a longer treatment period than hypertrophic cicatrices, independently from the volume and lasting of the injuries. Furthermore the injuries from burns and those located on the chest required applications which were more frequent and lasting for a longer period of time.

The treatment was interrupted if the results were estimated satisfactory by the subjects and that was corresponding to a 70-100 % reduction of the initial volume of the injuries. The length of the treatment was variable from 16 weeks to 30 months, depending on the above reported variables. During the follow-up (36 months) no subject was affected by injury recurrence. Both during the treatment and follow-up period local or systemic side-effects were not observed.

### Example 3

### Results of the topical treatment of burns by using the composition prepared according to example 1

The composition was applied topically in liquid form on n° 10 subjects affected by 1° and 2° superficial degree burns, occurred in the previous 24 hours, which extended over a mean cutaneous surface of about 25 cm² (varying from 4 minimum to 200 maximum cm²). The application of the composition caused in all the subjects a quick reabsorption of the liquid contained in the phlyctenas (within one hour after the first application), with reduction of the associated erythema and painful symptomatology. In all the subjects affected by 1° degree burns the restitutio ad integrum occurred two days after the injury event by a single application of the composition. In the subjects wherein phlyctenas were generated an immediate amelioration of the injury after the first application and restitutio ad integrum within 4/5 days occurred, during which period the composition was again applied to accelerate the exfoliation of the injured cutis. During the treatment local or systemic side-effects were not observed and the patients reported the application of the composition to be absolutely painless.

The present invention has been described for exemplary but not limiting purposes according to preferred embodiments thereof but it is to be understood that modifications and/or variations can be carried out by those skilled in the art without departing from the scope thereof as defined by the enclosed claims.

## Claims

1. Pharmaceutical composition for topical use comprising a mixture, in the suitable polymer, of trichloroacetic acid, 2-hydroxybenzoic acid, (1α, 2β, 5α)-5-methyl-2-(1-methylethyl)cyclohexanol and, if desired, other pharmaceutically acceptable adjuvants and excipients.

2. Pharmaceutical composition according to claim 1, wherein the polymer is selected from the group consisting of polyethylene glycols, polyvinyl alcohol, polyoxyethylene alcohol.

3. Pharmaceutical composition according to claim 2, wherein the polyethylene glycols are selected form the group consisting of tetraethylene glycol, hexaethylene glycol.

4. Pharmaceutical composition according to claims 1-3, wherein the concentration of trichloroacetic acid is from 20 % to 45 % w/v.

5. Pharmaceutical composition according to claim 4 wherein the concentration of trichloroacetic acid is about 25 % w/v.

6. Pharmaceutical composition according to claims 1-2, wherein the concentration of 2-hydroxybenzoic acid is from 10 % to 30 % w/v.

7. Pharmaceutical composition according to claim 6, wherein the concentration of 2-hydroxybenzoic acid is about 20 % w/v.

8. Pharmaceutical composition according to claims 1-7 further comprising at least one corticosteroid.

9. Pharmaceutical composition according to claim 8, wherein the corticosteroid is selected from the group consisting of triamcinolone, betamethasone, methylprednisolone and dexamethasone.

10. Pharmaceutical composition according to claim 9, wherein triamcinolone is contained at a concentration from 0,05 % to 20 %.

11. Pharmaceutical composition according to claim 10, wherein triamcinolone is contained at a concentration from 0,1 % to 10 %.

12. Pharmaceutical composition according to claims 1-11, wherein adjuvants and excipients are ethanol and sodium chloride.

13. Pharmaceutical composition according to claims 1-12 for use in the treatment of cutaneous injuries.

14. Pharmaceutical composition according to claims 1-12 for use in the treatment of cutaneous injuries resulting from mechanical traumas or surgical operations, bums, dermatitis both from animal sting and animal or poisonous plant contact.

15. Pharmaceutical composition according to claims 1-12 for use in the therapy and prevention of hypertrophic cicatrices and keloids.

16. Pharmaceutical composition according to claims 1-12 for use in aesthetic medicine.

17. Pharmaceutical composition according to claim 16 for use as exfoliating agent.

18. Pharmaceutical composition according to claims 1-17 in the form selected from the group consisting of ointment, gel, foam, liquid preparations, medicated plaster.

19. Process for the preparation of the pharmaceutical composition according to claims 1-18 comprising the following steps: a) prepare, in an anhydrous atmosphere, the distinct A and B mixtures, respectively, of (A) trichloroacetic acid at a concentration from 40 % to 90 % in the suitable polymer and (B) 2-hydroxybenzoic acid at a concentration from 20 % to 60 %, in the suitable polymer; b) mix, by adding small subsequent portions, same volumes of the two mixtures to obtain the A+B mixture; c) add a volume of a (1α, 2β, 5α)-5-methyl-2-(1-methylethyl)cyclohexanol saturated solution in anhydrous ethanol equal to 2 % of the volume of the A+B mixture; d) add sodium chloride in a such amount to obtain a final concentration of about 1,2 % w/v; e) keep the obtained composition in a bottle filled with anhydrous air at 30°C in reduced pressure conditions and leave at ambient temperature for about 24 hours.

20. Process according to claim 19, wherein the polymer is selected from the group consisting of polyethylene glycols, polyvinyl alcohol, polyoxyethylene alcohol.

21. Process according to claim 20, wherein the polyethylene glycols are selected form the group consisting of tetraethylene glycol, hexaethylene glycol.

22. Process according to claim 19-21, wherein to the mixture obtained in the step d) one or more corticosteroids is added.

23. Process according to claim 22, wherein the corticosteroids are selected from the group consisting of triamcinolone, betamethasone, methylprednisolone and dexamethasone.

24. Process according to claim 23, wherein triamcinolone is added in a such amount to obtain at a final concentration from 0,05 % to 20 %.

25. Process according to claim 24, wherein triamcinolone is added in a such amount to obtain at a final concentration from 0,1 % to 10 %.

## Patentansprüche

1. Pharmazeutische Zusammensetzung zur topischen Verwendung umfassend eine Mischung, befindlich in dem geeigneten Polymer, aus Trichloressigsäure, 2-Hydroxybenzoesäure, (1α, 2β, 5α)-5-Methyl-2-(1-methylethyl)cyclohexanol und, falls gewünscht, weiteren pharmazeutisch akzeptablen Hilfsstoffen und Arzneimittelträgern.

2. Pharmazeutische Zusammensetzung nach Anspruch 1, wobei das Polymer ausgewählt ist aus der Gruppe bestehend aus Polyethylenglykole, Polyvinylalkohol, Polyoxyethylenalkohol.

3. Pharmazeutische Zusammensetzung nach Anspruch 2, wobei die Polyethylenglykole ausgewählt sind aus der Gruppe bestehend aus Tetraethylenglykol, Hexaethylenglykol.

4. Pharmazeutische Zusammensetzung nach den Ansprüchen 1 - 3, wobei die Konzentration der Trichloressigsäure 20 % bis 45 % Gew./Vol. beträgt.

5. Pharmazeutische Zusammensetzung nach Anspruch 4, wobei die Konzentration der Trichloressigsäure ca. 25 % Gew./Vol. beträgt.

6. Pharmazeutische Zusammensetzung nach den Ansprüchen 1 - 2, wobei die Konzentration der 2-Hydroxybenzoesäure 10 % bis 30 % Gew./Vol. beträgt.

7. Pharmazeutische Zusammensetzung nach Anspruch 6, wobei die Konzentration der 2-Hydroxybenzoesäure ca. 20 % Gew./Vol. beträgt.

8. Pharmazeutische Zusammensetzung nach den Ansprüchen 1 - 7, ferner umfassend mindestens ein Kortikosteroid.

9. Pharmazeutische Zusammensetzung nach Anspruch 8, wobei das Kortikosteroid ausgewählt ist aus der Gruppe bestehend aus Triamcinolon, Betamethason, Methylprednisolon und Dexamethason.

10. Pharmazeutische Zusammensetzung nach Anspruch 9, wobei Triamcinolon in einer Konzentration von 0,05 % bis 20 % enthalten ist.

11. Pharmazeutische Zusammensetzung nach Anspruch 10, wobei Triamcinolon in einer Konzentration von 0,1 % bis 10 % enthalten ist.

12. Pharmazeutische Zusammensetzung nach den Ansprüchen 1 - 11, wobei Hilfsstoffe und Arzneimittelträger Ethanol und Natriumchlorid sind.

13. Pharmazeutische Zusammensetzung nach den Ansprüchen 1 - 12 zur Verwendung bei der Behandlung von Hautverletzungen.

14. Pharmazeutische Zusammensetzung nach den Ansprüchen 1 - 12 zur Verwendung bei der Behandlung von Hautverletzungen, die durch mechanische Verletzungen oder chirurgische Operationen, Verbrennungen, Dermatitis, durch den Stich eines Tieres oder auch durch den Kontakt mit einem Tier oder einer giftigen Pflanze hervorgerufen wurden.

15. Pharmazeutische Zusammensetzung nach den Ansprüchen 1 - 12 zur Verwendung bei der Therapie und Prävention von hypertrophischen Narben und Keloiden.

16. Pharmazeutische Zusammensetzung nach den Ansprüchen 1 - 12 zur Verwendung in der ästhetischen Medizin.

17. Pharmazeutische Zusammensetzung nach Anspruch 17 zur Verwendung als Abblätterungsmittel.

18. Pharmazeutische Zusammensetzung nach den Ansprüchen 1 - 17 in der Form, die aus der Gruppe bestehend aus Salbe, Gel, Schaum, flüssige Zubereitungen und medizinisch behandelte Pflaster ausgewählt ist..

19. Verfahren zur Herstellung der pharmazeutischen Zusammensetzung nach den Ansprüchen 1 - 18 umfassend die folgenden Schritte: a) Zubereiten der getrennten Mischungen A und B in einer wasserfreien Atmosphäre aus (A) Trichloressigsäure in einer Konzentration von 40 % bis 90 % in dem geeigneten Polymer und (B) 2-Hydroxybenzoesäure in einer Konzentration von 20 % bis 60 % in dem geeigneten Polymer; b) Mischen von gleicher Volumina der beiden Mischungen durch Zugabe kleiner, nachfolgender Portionen, um die A + B-Mischung zu erhalten; c) Zugabe eines Volumens einer gesättigten (1α, 2β, 5α)-5-Methyl-2-(1-methylethyl)cyclohexanol-Lösung in wasserfreiem Ethanol, welches gleichwertig mit 2 % des Volumens der A + B-Mischung ist; d) Zugabe von Natriumchlorid in einer solchen Menge, dass eine Endkonzentration von ca. 1,2 % Gew./Vol. erhalten wird; e) Aufbewahren der erhaltenen Zusammensetzung bei 30° C unter reduziertem Druck in einer Flasche, die mit wasserfreier Luft gefüllt ist, und Stehenlassen bei Raumtemperatur für ca. 24 Stunden.

20. Verfahren nach Anspruch 19, wobei das Polymer ausgewählt ist aus der Gruppe bestehend aus Polyethylenglykole, Polyvinylalkohol, Polyoxyethylenalkohol.

21. Verfahren nach Anspruch 20, wobei die Polyethylenglykole ausgewählt sind aus der Gruppe bestehend aus Tetraethylenglykol, Hexaethylenglykol.

22. Verfahren nach den Ansprüchen 19 - 21, wobei zu der Mischung erhalten im Schritt d) eines oder mehrere Kortikosteroide zugegeben werden.

23. Verfahren nach Anspruch 22, wobei die Kortikosteroide ausgewählt sind aus der Gruppe bestehend aus Triamcinolon, Betamethason, Methylprednisolon und Dexamethason.

24. Verfahren nach Anspruch 23, wobei das Triamcinolon in einer solchen Menge zugegeben wird, um eine Endkonzentration von 0,05 % bis 20 % zu erhalten.

25. Verfahren nach Anspruch 24, wobei das Triamcinolon in einer solchen Menge zugegeben wird, um eine Endkonzentration von 0,1 % bis 10 % zu erhalten.

## Revendications

1. Composition pharmaceutique pour utilisation topique comportant un mélange, dans le polymère approprié, d'acide trichloroacétique, d'acide 2-hydroxybenzoïque de (1α,2β,5α)-5-méthyl-2-(1-méthyléthyl)-cyclohexanol et, si cela est souhaité, d'autres adjuvants et excipients acceptables sur le plan pharmaceutique.

2. Composition pharmaceutique selon la revendication 1, dans laquelle le polymère est sélectionné dans le groupe composé de polyéthylèneglycols, de poly(alcool vinylique) et d'alcool de polyéthoxylé.

3. Composition pharmaceutique selon la revendication 2, dans laquelle les polyéthylèneglycols sont sélectionnés dans le groupe composé du tétraéthylèneglycol et de l'hexaéthylèneglycol.

4. Composition pharmaceutique selon les revendications 1-3, dans laquelle la concentration en acide trichloroacétique est de 20 % à 45 % en poids/volume.

5. Composition pharmaceutique selon la revendication 4, dans laquelle la concentration en acide trichloroacétique est d'environ 25 % en poids/volume.

6. Composition pharmaceutique selon les revendications 1-2, dans laquelle la concentration en acide 2-hydroxybenzoïque est de 10 % à 30 % en poids/volume.

7. Composition pharmaceutique selon la revendication 6, dans laquelle la concentration en acide 2-hydroxybenzoïque est d'environ 20 % en poids/volume.

8. Composition pharmaceutique selon les revendications 1-7, comprenant en outre au moins un corticostéroïde.

9. Composition pharmaceutique selon la revendication 8, dans laquelle le corticostéroïde est sélectionné dans le groupe composé de la triamcinolone, de la bêtaméthasone, de la méthylprednisolone et de la dexaméthasone.

10. Composition pharmaceutique selon la revendication 9, dans laquelle la triamcinolone est contenue dans une concentration de 0,05 à 20%.

11. Composition pharmaceutique selon la revendication 10, dans laquelle la triamcinolone est contenue dans une concentration de 0,1 % à 10 %.

12. Composition pharmaceutique selon les revendications 1-11, dans laquelle les adjuvants et excipients sont l'éthanol et le chlorure de sodium.

13. Composition pharmaceutique selon les revendications 1-12, pour une utilisation dans le traitement de lésions cutanées.

14. Composition pharmaceutique selon les revendications 1-12, pour une utilisation dans le traitement de lésions cutanées résultant de traumatismes mécaniques ou d'opérations chirurgicales, de brûlures, de dermites dues à une piqûre d'animal et à un contact avec un animal ou une plante irritante.

15. Composition pharmaceutique selon les revendications 1-12, pour une utilisation dans le traitement et la prévention de cicatrices hypertrophiques et de chéloïdes.

16. Composition pharmaceutique selon les revendications 1-12, pour une utilisation en médecine esthétique.

17. Composition pharmaceutique selon la revendication 16, pour une utilisation en tant qu'agent exfoliant.

18. Composition pharmaceutique selon les revendications 1-17 dans la forme sélectionnée dans le groupe composé d'un onguent, d'un gel, d'une mousse, de préparations liquides et de pansement médicamenteux.

19. Procédé de préparation de la composition pharmaceutique selon les revendications 1-18, comportant les étapes suivantes : a) préparation, dans une atmosphère anhydre, des mélanges distincts A et B, respectivement de (A) acide trichloroacétique à une concentration de 40 % à 90 % dans le polymère approprié, et de (B) acide 2-hydroxybenzoïque à une concentration de 20 % à 60 % dans le polymère approprié ; b) mélange, par ajout de petites portions successives, de volumes identiques des deux mélanges, pour obtenir le mélange A+B, ; c) ajout d'un volume d'une solution saturée de (1α,2β,5α)-5-méthyl-2-(1-méthyléthyl)cyclohexanol dans de l'éthanol anhydre égal à 2 % du volume du mélange A+B ; d) ajout de chlorure de sodium dans une quantité permettant d'obtenir une concentration finale d'environ 1,2 % en poids/volume ; e) conservation de la composition obtenue dans une bouteille remplie d'air anhydre à 30 °C et dans des conditions de pression réduite, et conservation à la température ambiante pendant environ 24 heures.

20. Procédé selon la revendication 19, dans lequel le polymère est sélectionné dans le groupe composé de polyéthylèneglycols, de poly(alcool vinylique) et d'alcool de polyéthoxylé.

21. Procédé selon la revendication 20, dans lequel les polyéthylèneglycols sont sélectionnés dans le groupe composé du tétraéthylèneglycol et de l'hexaéthylèneglycol.

22. Procédé selon les revendications 19-21, dans lequel un ou plusieurs corticostéroïdes sont ajoutés au mélange obtenu à l'étape d).

23. Procédé selon la revendication 22, dans lequel les corticostéroïdes sont sélectionnés dans le groupe composé de la triamcinolone, de la bêtaméthasone, de la méthylprednisolone et de la dexaméthasone.

24. Procédé selon la revendication 23, dans lequel la triamcinolone est ajoutée dans une quantité permettant d'obtenir une concentration finale de 0,05 à 20 %.

25. Procédé selon la revendication 24, dans lequel la triamcinolone est ajoutée dans une quantité permettant d'obtenir une concentration finale de 0,1 à 10 %.
